# EUROPEAN PATENT APPLICATION

(11) **EP 4 606 409 A1**
(43) Date of publication of application: **27.08.2025**
(21) Application number: 22826123.6
(22) Date of filing: 18.10.2022
(51) Int. Cl.: A61M 16/04

(54) **ENDOTRACHEAL TUBE ASSEMBLY**

(71) Applicant: BRAVO GARCÍA, Pedro Luis, 38001 Santa Cruz de Tenerife (ES)
(72) Inventor: BRAVO GARCÍA, Pedro Luis, 38001 Santa Cruz de Tenerife (ES)
(74) Representative: Isern Patentes y Marcas S.L.
(86) International application number: PCT/ES2022/070658
(87) International publication number: WO 2024/084106

(57) **Abstract**

The assembly comprises: an endotracheal tube (10), with an external wall (11); and a suction channel (12), with a first more distal portion (13), equipped with a suction end (14), and externally attached to the external wall (11). The suction end (14) is guaranteed to occupy the supraepiglottic area of a patient (1), above the vocal cords (3), larynx (4), and epiglottis (5). The suction channel (12) has a banana-shaped cross-section, with two opposite curved segments (15, 16). The suction channel (12) has orifices (21) in the vicinity of the suction end (14). The suction end (14) ends in a bevel (22). A supraglottic barrier (described in WO 2021/234183 (BRAVO) (17) may be housed in the endotracheal tube (10). The invention allows suctioning performance to be improved, as well as reducing damages to the patient.

## Description

### TECHNICAL FIELD

The present invention can be included within the field of instruments and devices for use in mechanical ventilation of patients. In particular, the object of the invention relates to an endotracheal tube assembly.

### BACKGROUND OF THE INVENTION

Currently, at the end of anaesthesia, those secretions from the oral or pharyngeal cavity are suctioned, prior to extubating the patient to avoid bronchial suctioning, laryngospasm and bronchospasm. In a patient intubated for a longer time, these secretions should be suctioned continuously or intermittently in an amount of 500 to 1500 ml in 24 hours under normal conditions (the volume can be even greater in pathological cases, for example intestinal obstruction). Another source of secretions are fluids coming from reflux from the stomach or from the oesophagus.

The aim of the referred suctioning is to avoid bronchial suctioning, i.e., entry of any type of secretion into the airways, which would produce bronchospasm, laryngeal spasm, cough or, in more complicated cases, pneumonia in the days following the surgical procedure that has required anaesthesia.

The following two methodologies are currently used in anaesthesia to remove secretions.

According to a first methodology, suctioning is performed blindly, inserting a probe and suctioning until no more secretions are obtained. This methodology does not guarantee that all the content will be suctioned and, therefore, and as residue remains, laryngospasm, bronchial suctioning, bronchospasm and cough will occasionally occur as soon as the anaesthesia is reversed and the patient recovers all their reflexes. Another negative effect of current blind suctioning is the production of small lacerations in the mucosal wall due to the adhesion of the probe, which is known as the wall effect, which causes damage to the structures to which the probe adheres, producing odynophagia and, in addition, reduces suctioning effectiveness. Currently, in anaesthesia, most of these procedures are blind.

According to a second methodology, suction-assisted laryngoscopy is performed under direct vision. This requires a high anaesthetic depth, since it is one of the manoeuvres that most triggers known sympathetic activation. It is at the end of anaesthesia when the patient must be at their shallowest from the anaesthetic point of view, since we are "waking up" the patient. By deepening the anaesthesia for this manoeuvre we lengthen the time during which the patient is under the effects of anaesthesia.

In certain cases, during anaesthesia, reflux and vomiting occur that must be suctioned immediately and in the appropriate place. If the flow is very low, it inadvertently produces bronchial suctioning and if it is too high, the suctioning is not enough.

In patients intubated for longer than the duration of anaesthesia (for example, in intensive care patients), endotracheal tubes with a suction channel below the vocal cords are used to reduce the passage of the aforementioned secretions, and thus, obviously, reduce the incidence of mechanical ventilation-associated pneumonia (without eliminating the possibility of its occurrence). This channel is found in the thickness of the endotracheal tube, greatly diminishing its capacity and limiting its use only to non-reinforced tubes.

The suction channel was a great advance. Current devices have the suction point in the subglottic space, the logical place thereof, below the vocal cords and in what is known as the elastic cone, just above the balloon that is placed in the trachea, which is the common point through which any secretion entering the respiratory tree must pass. This obviously pushes to place any suctioning in this place. Another site implied that some secretions would be allowed to pass.

However, the site where the secretions accumulate the most is in the oropharynx and not in the previously described area (at the level of the elastic cone), as it is such a small space that sometimes it is no more than 1.5 cm. It is at this point that all the nerves that innervate the complicated phonation apparatus, in particular, eleven of the twelve muscles involved in phonation and swallowing, as well as in the defense against bronchial suctioning, enter. Negative (even slight) pressure on these nerves causes damage, either permanently or temporarily.

For this reason, the applicant states that, in the current technique, secretions are being suctioned at the worst possible site from the anatomical and physiological point of view, even if the site is obvious, a high price is paid in damage to the vocal cords and to the swallowing. Suctioning normally requires very high negative pressures, ranging from 40 to 100 mm of mercury and, consequently, an increase in vocal cord injuries is observed, although it can effectively diminish the incidence of ventilator-associated nosocomial pneumonia, but without completely eliminating it. The devices to date have been causing this so frequent mechanism of injury, especially in recent years (its incidence greatly increased in the recent Covid epidemic). Moreover, it is in this area where the main arteries, veins and lymphatics that irrigate and drain the larynx enter and leave. Therefore, a totally different and novel approach is required to avoid such injuries and ensure that suctioning is always carried out in the best possible area and with a much greater absorption capacity.

According to current solutions, described above, although it is the most obvious area for suctioning, a high price is paid in terms of voice and swallowing damage with the current technique.

In the current state of the art, the suction channel, which has a circular cross section, is housed inside the wall of the same endotracheal tube (intramural suction channel), which limits the suctioning capacity thereof, as it has a small diameter (it clogs more easily), while weakening the strength of the same tube by having a partially hollow wall. This forces the current endotracheal tubes to have a greater hardness, which contributes to an increase in damage to the body structures that are, in use of the tube, adjacent to said tube. In addition, it limits its use only to non-reinforced tubes, since in a reinforced one the thickness does not allow it.

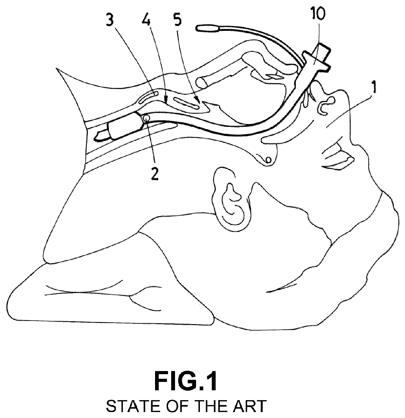

Figure 1 shows the anatomical location of the place where suctioning is currently performed in a patient (1) by means of an intramural suction channel (2), which is disadvantageously very close to the vocal cords (3), larynx (4) and epiglottis (5).

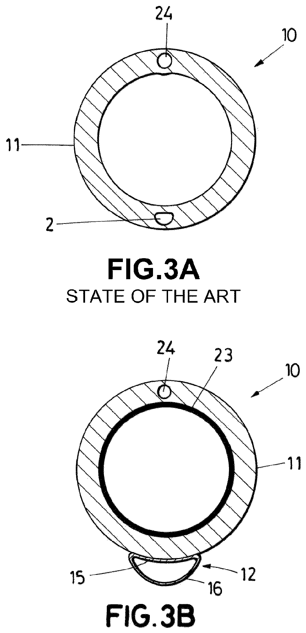

For its part, figure 3A, also referring to the state of the art, shows a cross section of the endotracheal tube assembly in the state of the art, with intramural channel (2).

### DESCRIPTION OF THE INVENTION

The present invention solves the aforementioned problem, by means of an endotracheal tube assembly combining five novel aspects: 1) adjoining suction channel; 2) novel banana shape of the suction channel according to the independent claim, which guarantees a suctioning capacity in any of the situations that may arise in these patients, 3) lower hardness than the endotracheal tube; 4) the correct place for suctioning secretions, secured in the supra-epiglottic area, as it is fixed. The suction channel is located above the vocal cords, the larynx, and the epiglottis, unlike with current channels that are below the vocal cords and therefore are subglottic, since it is the meeting point where all secretions reach before passing to the respiratory tree. 5) A use of the suction channel in conjunction with the supraglottic barrier (described in WO 2021/234183 (BRAVO) prevents the risk of wall effect when suctioning and allows secretions to be suctioned in a higher area; it isolates the epiglottic space, protecting the vocal cords.

The present invention limits, in particular, the aforementioned risk and incidence of spasms (laryngospasm, bronchospasm), odynophagia, oropharyngeal injuries, and cough after recovery from anaesthesia, due to non-suctioned residual secretions.

The invention also limits the lacerations caused by the aforementioned wall effect.

Furthermore, the present invention also limits suctioning damage to the nerves for phonation and swallowing.

Until now, experts would never suggest suctioning above the epiglottis because, according to this concept, it would not be guaranteed that secretions continue to pass to the respiratory tree or tract; in addition, the aforementioned wall effect would occur. Exclusively changing the place would go in the opposite direction to the state of the art.

In this invention, the suction channel is adjoined to, and independent from, the endotracheal tube, instead of passing through the inside of the wall of said endotracheal tube. Thus, the suction channel can have a larger diameter, since it will not be limited by the wall of the endotracheal tube, which allows much larger amounts of secretions and higher viscosity to be suctioned.

Using the endotracheal tube assembly of the invention, in collaboration with a supraglottic balloon, for example, such as that which is disclosed in WO 2021/234183 (BRAVO), improves the performance of both, since the supraglottic balloon: isolates the subglottic space from the arrival of secretions; further reduces the risk of the wall effect; allows secretions to be suctioned in an upper area; isolates the epiglottic space, protecting the vocal cords.

The solutions presented by the invention are based on the combination of a new morphology of a banana-shaped suction channel; in a different place; fixed; adhered with a lower hardness to the endotracheal tube and associated with WO 2021/234183 (BRAVO), which was previously not accessible.

### BRIEF DESCRIPTION OF THE DRAWINGS

As a complement to the description, and for the purpose of helping to make the features of the invention more readily understandable, in accordance with a preferred practical exemplary embodiment thereof, said description is accompanied by an assembly of drawings constituting an integral part of the same, which by way of illustration and not limitation, the following has been represented:
Figure 1 shows an image of a patient intubated with an endotracheal tube equipped with an intramural suction channel according to the state of the art.
Figure 2 shows an image of the patient of Figure 1, intubated with an endotracheal tube assembly according to the present invention.
Figures 3A and 3B show respective cross sections of the endotracheal tube assembly in the state of the art (figure 3A) and compared with the invention (figure 3B).
Figure 4 shows a detailed view of the distal end of the suction channel and the relationship thereof with the supra-epiglottic barrier.
Figure 5 shows a detailed view of the proximal end, with a distal connector with a 45° suction window and universal connection to any suction source.

### PREFERRED EMBODIMENT OF THE INVENTION

With the aid of figures 1-5 referred to above, a detailed description of a preferred embodiment of an endotracheal tube assembly according to the present invention is provided below.

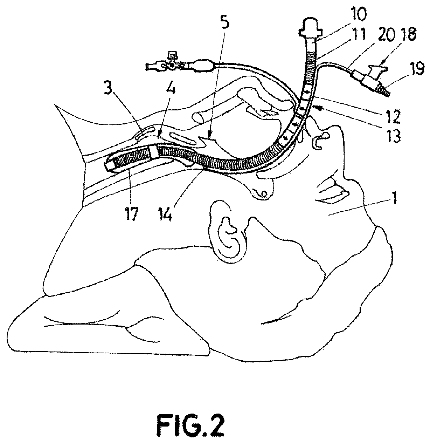

According to that which is shown in figure 2, the assembly of the invention comprises an endotracheal tube (10), as is known in the state of the art, and which comprises an external wall (11). The assembly further includes a suction channel (12), having a first (more distal) portion (13), linked to the endotracheal tube (10), wherein said first portion (13) includes a more distal suction end (14), through which the secretions enter the suction channel (12). Advantageously, the first, most distal portion (13) of the suction channel (12) is fixed, such as adhered, externally along the external wall (11) of the endotracheal tube (10). The suction channel (12) is preferably located in such a way that in use it occupies an upper position along the craniocaudal axis in the patient (1).

Additionally, the first portion (13) of the suction channel (12) is arranged in such a way that the suction end (14) is preferably arranged in a position of the endotracheal tube (10) which, in use, corresponds to the supra-epiglottic area in the patient (1), i.e., above the vocal cords (3), the larynx (4), and the epiglottis (5), an area with a large space. Figure 1 contrasts with the site of the current state (2) of the art where the damage being described is produced, as it is the anatomical area where the nerves of the larynx (4), the main vessels, enter, and where the main veins and lymphatics of the larynx (4) start from, being an area with a very reduced space. In addition, it is a channel with low capacitance and easy obstruction, as it is intramural.

Figure 3B shows that, preferably, the suction channel (12) has a so-called "banana" cross-section, formed by two opposite curved segments (15, 16) of different curvature: an internal segment (15), with circular curvature equivalent to the curvature of the endotracheal tube (10), and an external segment (16) with a smaller curvature than the internal segment (15) and which is also preferably, although not necessarily, circular. The internal segment (15) and the external segment (16) converge at the ends of the internal segment (15). Therefore, by having said banana shape, the suction channel (12) has a cross section that greatly increases the suction capacity and prevents more viscous secretions from obstructing it, compared to the intramural suction channel (2) of the state of the art represented in figure 3A.

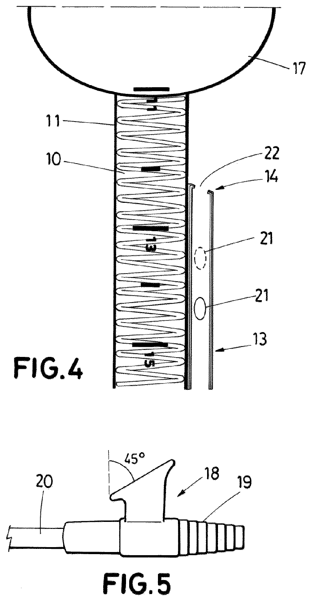

The endotracheal tube (10) can optionally be associated with a supraglottic balloon (17), see figures 2 and 4, which preferably corresponds to the supraglottic barrier described in WO 2021/234183 A1 (BRAVO), in which case, it is guaranteed that the suctioning that has to be performed at any time during anaesthesia, or in patients (1) intubated for long periods in intensive care units, is performed in the appropriate site, with sufficient suctioning capacity, and preventing damage to the mucous membranes and structures of the patient (1). Figure 2 shows, in contrast to figure 1, the arrangement of the assembly of the invention in an intubated patient (1).

At its proximal end, opposite the suction end (14), the suction channel (12) is fixed to a connector (18), see figure 5, which can be a universal connector (18) for connecting to any suction connection, see figures 2 and 5, which allows, on the one hand, its connection to a suction source (not shown) and, on the other hand, optionally modulating an activation or deactivation of the suctioning with a finger, wherein in this case the angle to place the finger is 45°, reducing the possibility of user contamination when using the suction channel (12). The connector (18), in particular, the universal connector (18), is generally configured to be connected to the system of any operating room or intensive care unit, for which it preferably has a conical shape, with an external grading (19), and with a minimum internal diameter of 4 mm and at least 7 mm at the point of smallest external diameter, progressing from this point gradually until reaching 9.5 or 10 mm of external diameter. This connection and, in particular, the quoted dimensions, can be independent of the type of endotracheal tube (10) used. This connection allows both continuous and intermittent suctioning.

The suction channel (12) has a length that in turn depends on the dimensions of the endotracheal tube (10). For example, for an endotracheal tube (10) with an internal diameter of 7 mm, the suction channel (12) can have a total length of about 38-40 cm starting from the suction end (14), located in a more distal position. Approximately the first third, around the first 12-13 centimetres, from the suction end (14), runs fixed (such as adhered, welded, etc.) to the endotracheal tube (10), in a position which, in the event of a (supra)epiglottic balloon (17) being arranged, it is approximately 1-3 cm, for example, 1.5 cm, away from the closest portion of said supraglottic balloon (17).

The suction channel (12) comprises, see figure 2, a second, more proximal, portion (20), following the first portion (13), and which is separated from the external wall (11) of the endotracheal tube (10). As a guideline, for an endotracheal tube (10) with an internal diameter of 7 mm, the first portion (13) is approximately 25 cm long. The first portion (13) ends at an attachment point for attaching (such as welding, adhering, etc.) to the endotracheal tube (10), which allows the second portion (20) to start, with respect to the first portion (13), with an angle not greater than 25°, in order, on the one hand, to avoid tearing in the fixation, whether adhesive, or welded, etc., of the first portion (13) and, on the other hand, to prevent kinking of the suction channel (12) during handling of the assembly of the invention.

The dimensions of the cross section of the suction channel (12) depend in turn on the cross section of the endotracheal tube (10). For a case in which both the internal segment (15) and the external segment (16) are circular, and with an endotracheal tube (10) with an internal diameter of 7 mm, approximate values of 1, 2 mm internal diameter are proposed for the internal segment (15), as well as 4 mm internal diameter for the external segment (16).

Again, as an example, for a 7 mm diameter tube, the internal dimensions of the suction channel (12) are 6 mm long and 4 mm wide in a banana shape. The absolute dimensions vary depending on the size of the endotracheal tube (10), which includes adult and paediatric sizes. These dimensions of the suction channel (12) are in the first portion (13) thereof, which is adhered to the endotracheal tube (10).

According to figure 4, in the vicinity of the suction end (14), for example, at about 10 mm, the suction channel (12) has at least one orifice (21), preferably two lateral orifices (21) opposed to each other, the dimensions of which depend on the diameter of the endotracheal tube (10). The orifices (21) are countersunk and are spaced apart between their closest points by a distance of between 3 and 10 mm, for example 5 mm. The presence of the orifice (21) or orifices (21), on the one hand, increases the suction capacity, due to Poiseuille's law, and also avoids loss of function, in case of obstruction of the suction end (14).

At the suction end (14), the suction channel (12) ends in a bevel (22), for example, at 60°, to prevent collapsing. Likewise, figure 4 shows the relationship with the supraglottic barrier (17) described in WO 2021/234183 (BRAVO). In this sense, figures 3A and 3B illustrate respective vacuum channels (24), preferably intramural, used to produce and release pressure in accordance with the operation of the supraglottic barrier (17).

The presence of the orifices (21), as well as the banana shape of the cross section of the suction channel (12), and the bevel (22), increase the capacity for suctioning liquids depending on the viscosity thereof. In the case of physiological saline, water, saliva, or reflux of gastric secretions, more than 1 liter can be absorbed in just 15 seconds in an endotracheal tube (10) with an internal diameter of 7 to 8 mm, which is impossible in the current state of the art.

Due to its supraglottic (or supraepiglottic) arrangement, the dimensions of the suction channel (12), although it is arranged outside the endotracheal tube (10), allow the assembly not to interfere in the narrowest area through which the endotracheal tube (10) would pass, which are the vocal cords (3). In addition to greatly simplify the manufacture thereof, it has better features, by increasing the size of the cross section as required. This improves the absorption capacity by more than 70% compared to a catheter or a channel of those which are currently used, also surprisingly reducing the resistance to suctioning, up to 90%, compared to the current technique, due to the "banana" shape of the cross section of the suction channel (12).

The "banana" shape described serves to reduce the "decubitus" effect (damage to structures due to sustained support); furthermore, being of a much lower hardness than the endotracheal tube (10), it serves to additionally minimise the referred decubitus effect. The banana shape, as opposed to the circular section currently used in suction catheters, leads to a more efficient distribution of bearing pressure. The shape allows it to adhere to the endotracheal tube (10) without being part of said endotracheal tube (10). It needs this shape to be able to suction in the required quantities with a much higher suction capacity than the current channel inside the endotracheal tube (10). This shape also allows better tolerance in the pharynx of patients (1) once the endotracheal tube (10) has been inserted, since the tangential pressure on the walls of the pharynx is much lower. As it is fixed, there is no friction or shearing. The shape requirement is also due to the fact that this invention can be used in any type of endotracheal tube (10), whether ringed; reinforced or not reinforced. Figure 3B shows the joint use with an endotracheal tube (10), which has reinforcement rings (23).

Figure 3B shows a cross-section where the relationship of the suction channel (12) and its morphology is displayed in relation to the endotracheal tube (10). Said arrangement allows the suction channel (12) to be attached to any type of endotracheal tube (10), whether reinforced or non-reinforced, with one lumen or with different lumens. The current technique uses the aforementioned intramural suction channel (2), see figure 3A, greatly reducing the suction capacity and weakening the tube wall. In the opposite direction, the suction channel (12) of the invention is attached to the endotracheal tube (10), with a larger section and, therefore, greater suction capacity.

Since the endotracheal tube (10) according to the present invention can be inserted up to the reference of the vocal cords (3), it is guaranteed that the suction channel (12) is at a suitable distance, which in turn is a function of the age of the patient. For example, for an endotracheal tube (10) with an internal diameter of 7 mm, the suction channel (12) is advantageously, on the one hand, at a non-dangerous distance from the vocal cords (at about 7 cm) and, on the other hand, is located at the site where the largest secretions accumulate.

When the assembly of the invention is used together in relation to (supra) epiglottic balloons (17), said balloons (17) keep said suction channel (12) isolated from the walls of the oropharynx, thereby allowing better suctioning and preventing it from being obstructed due to the aforementioned wall effect.

Likewise, the suction channel (12) can also be present in endotracheal tubes (10) deprived of said balloon (17), which at least guarantees an optimal location for the suctioning, although there is no guarantee that the wall effect will not occur, nor will the airway be completely isolated.

By resolving bronchial suctioning, the present invention greatly reduces mechanical ventilation-associated pneumonia, much more effectively than the current technique of subglottic suction channels; as well as, contrary to the current technique, doing it without damaging the nerves or blood vessels that innervate and irrigate the larynx (4).

The assembly of the invention allows much larger contents to be suctioned than with the intramural suction channel (2) of current devices. Given the savings that this entails in terms of ventilation-associated pneumonia and the damage caused, in many cases permanently, it has reasonable guarantees of success.

The present invention can be used industrially, because the changes are made at the end, once the endotracheal tube (10) has been extruded, so it does not represent a problem for its industrial production, nor great changes in the production line of any endotracheal tube (10).

The dimensions and arrangement of both the endotracheal tube (10), and the suction channel (12) and the components thereof, can vary depending on the morphology of the patients, distinguishing in particular its use for adult patients and for paediatric patients, with appropriate modifications.

### List of elements

- 1: patient
- 2: intramural suction channel
- 3: vocal cords
- 4: larynx
- 5: epiglottis
- 10: endotracheal tube
- 11: external wall
- 12: external suction channel
- 13: first portion
- 14: suction end
- 15: internal segment
- 16: external segment
- 17: supraglottic barrier (described in WO 2021/234183 (BRAVO)
- 18: connector
- 19: external grading
- 20: second portion
- 21: orifice
- 22: bevel
- 23: Reinforcing ring
- 24: Vacuum channels

## Claims

1. An endotracheal tube assembly, comprising:
- an endotracheal tube (10), with an external wall (11); and
- a suction channel (12), with a first, more distal, portion (13), equipped with a suction end (14), for suctioning secretions;
**characterised in that** the first portion (13) of the suction channel (12) is externally attached along the external wall (11) of the endotracheal tube (10).

2. The endotracheal tube assembly according to claim 1, **characterised in that** the suction channel (12) is located in such a way that, in use, occupies an upper position in a patient (1) along the craniocaudal axis of the patient (1).

3. The endotracheal tube assembly according to any of claims 1-2, **characterised in that** the first portion (13) of the suction channel (12) is arranged in such a way that the suction end (14) is located in a position of the endotracheal tube (10) which, in use, corresponds to the supra-epiglottic area in the patient (1), i.e., above the vocal cords (3), the larynx (4), and the epiglottis (5).

4. The endotracheal tube assembly according to any of claims 1-3, **characterised in that** the suction channel (12) has a banana-shaped cross-section, formed by two opposite curved segments (15, 16) of different curvature: an internal segment (15), with circular curvature equivalent to the curvature of the endotracheal tube (10); and an external segment (16), with a smaller curvature than the internal segment (15) and which is preferably also circular, the internal segment (15) and the external segment (16) converging at the ends of the internal segment (15).

5. The endotracheal tube assembly according to any of claims 1-4, **characterised in that** the suction channel (12) comprises a second, more proximal, portion (20), following the first portion (13), and which is separated from the external wall (11) of the endotracheal tube (10).

6. The endotracheal tube assembly according to claim 5, **characterised in that** the first portion (13) ends at an attachment point for attaching to the endotracheal tube (10) which allows the second portion (20) to start, with respect to the first portion (13), with an angle not greater than 25°.

7. The endotracheal tube assembly according to any of claims 1-6, **characterised in that** the suction channel (12) has at least one orifice (21), preferably two opposite lateral orifices (21), in the vicinity of the suction end (14).

8. The endotracheal tube assembly according to any of claims 1-7, **characterised in that** the suction channel (12) ends with a bevel (22) at the suction end (14).

9. The endotracheal tube assembly according to any of claims 1-8, **characterised in that** it additionally comprises a supraglottic barrier (17) housed in the endotracheal tube (10).
